# EUROPEAN PATENT SPECIFICATION

(11) **EP 3 921 008 B1**
(45) Date of publication and mention of the grant of the patent: **22.03.2023**
(21) Application number: 20705783.7
(22) Date of filing: 31.01.2020
(51) Int. Cl.: A61M 16/20, A61M 16/00, A61M 16/22, A61M 16/16

(54) **A VALVE ACTUATION ASSEMBLY AND A SYSTEM FOR CONTROLLING A SUPPLY OF GAS FOR INHALATION BY A USER**
VENTILBETÄTIGUNGSANORDNUNG UND SYSTEM ZUR STEUERUNG DER ZUFUHR VON GAS ZUR INHALATION DURCH EINEN BENUTZER
ENSEMBLE D'ACTIONNEMENT DE VALVE ET SYSTÈME DE COMMANDE D'ALIMENTATION EN GAZ POUR INHALATION PAR UN UTILISATEUR

(30) Priority: 04.02.2019 GB 201901526; 27.11.2019 GB 201917268
(43) Date of publication of application: 15.12.2021
(73) Proprietor: Camcon Medical Limited, Cambridge, Cambridgeshire CB24 9NL (GB)
(72) Inventor: WYGNANSKI, Wladyslaw, Cambridge Cambridgeshire CB4 0WS (GB); POTTER, Charles, Cambridge Cambridgeshire CB4 0WS (GB)
(74) Representative: Sharrock, Daniel John
(86) International application number: PCT/GB2020/050229
(87) International publication number: WO 2020/161471

(56) References cited:
- WO-A2-2013/040198
- BE-A- 645 564
- US-A1- 2005 039 749
- US-A1- 2014 290 659
- US-A1- 2016 220 780

## Description

### Field of the invention

The present invention relates to a system for providing gas for inhalation by a user comprising a valve actuation assembly. More particularly, it may provide assemblies and systems suitable for use in controlling the delivery of air to a person suffering from sleep apnoea or a person requiring ventilator support in a hospital or home environment.

### Background to the invention

Sleep apnoea is one of the most common sleep disorders. It is characterised by frequent episodes of upper airway collapse during sleep. Increasingly, obstructive sleep apnoea (OSA) is recognised as an independent risk factor for several clinical consequences, including systemic hypertension, cardiovascular disease, stroke and abnormal glucose metabolism. There are currently an estimated 936 million people suffering from OSA globally.

A known treatment for OSA requires the use of a Continuous Positive Airway Pressure (CPAP) device. This involves wearing a mask overnight and a bedside unit provides positive air pressure within the mask throughout the night at a pressure of around 4 to 25cm H₂O. However users often feel it is difficult to breathe because of the positive pressure. More sophisticated devices gradually ramp up the pressure from a low level as the patient goes to sleep so that it is less noticeable when the patient first gets into bed.

A more expensive device provides Bi-Level Positive Airway Pressure (BiPAP). This reduces the supplied air pressure to around 4cm H₂O when the patient breathes out and increases it up to 25cm H₂O during inhalation.

CPAP devices are usually attached to a face mask or nasal mask via a wide bore tube (with a diameter of around 2cm). The wide tube ensures that the airflow is not restricted and that exhaled CO₂ is washed out from the mask.

Existing masks have an exhaust port that is continuously open to the atmosphere, through which excess air is vented continually. This air venting causes a continuous flow of air across the face which can be uncomfortable and disturbing for some patients.

US2016/220780A1 discloses an opening and closing device for a mask vent valve. WO2013/040198A2 discloses a control system providing automated control of gas washout of a patient interface, such as a mask or nasal prongs. BE645564A discloses a control valve for artificial respiration apparatus which operates in response to the patient's attempts to breathe. US2005/039749A1 discloses an insufflation-exsufflation system for removal of broncho-pulmonary secretions. US2014/290659A1 discloses a reversing valve for reversing a fluid flow to a patient.

### Summary of the disclosure

The present invention provides a system for controlling a supply of gas for inhalation by a user as defined by the appended independent claim 1.

When the gas vent valve is closed, gas from the supply outlet is inhaled by the user. During exhalation, the gas vent valve can be opened so that the patient breathes out easily, rather than against a positive pressure. By using such a system, a continuously open exhaust port may not be required on the user's mask, thereby avoiding the associated disadvantages. This makes use of the system more comfortable for the user.

The supply outlet can supply pressurised gas to the user during inhalation. Alternatively, gas may be drawn from the system by the action of the user's lungs.

In preferred examples, the system may include a gas pressure reduction device coupled to a vent gas flow path carrying exhaled gas from a user to reduce the gas pressure in the vent gas flow path between the user and the device. The gas pressure reduction device may be in the form of a pump or a fan for example. It may reduce the gas pressure in the vent gas flow path between the user and the device so as to make it easier for a user to exhale. Preferably, the gas pressure reduction device is located downstream of the gas vent valve.

According to the invention, the actuator is an electromagnetic actuator comprising: an armature comprising a permanent magnet; two coils; and two coil cores, each of which extends within a respective coil; wherein the armature is moveable relative to the coils between first and second stable rest positions by passing a current through at least one of the coils, and in each rest position, the armature is closer to one of the coil cores than the other of the coil cores and is spaced by a gap from the closer coil core.

Accordingly, the armature may not contact a coil core (and preferably is not brought into contact with any other part of the actuator) as it moves into each rest position. This avoids the generation of a clicking noise as the armature switches into either rest position. The switching of the actuator may therefore be substantially silent. In some existing actuators, an armature is accelerated into contact with a coil core in each stable rest position, causing a loud clicking noise to be produced. In the present application, frequent generation of loud clicking sounds is likely to be disturbing for a patient.

The actuator may have only two stable rest positions and therefore constitute a bistable actuator. In some embodiments, there may be two end-of-travel positions which are stable rest positions. In some cases, there may also be one or more further stable rest positions located between the two end-of-travel positions which may be selected by suitable control of the actuator.

The actuator preferably includes two resilient components, wherein a respective one of the resilient components is compressed by movement of the armature towards each stable rest position (or more particularly, in some embodiments, during the final part of the movement of the armature towards and into each stable rest position). The resilient components may thereby arrest motion of the armature and so avoid a contact which generates a clicking noise.

The resilient component may be able to store potential energy as the armature moves into each of its first and second rest positions. When the armature is switched towards its other position, this potential energy is released and acts to accelerate the armature towards the other position. The resilient component may be in the form of a single resilient part or alternatively it may be an assembly of parts.

Preferably, the system includes a gas supply valve for opening and closing a gas flow path to the supply outlet. The gas supply valve may be operated so that gas is only supplied to the user when required for inhalation. The gas may be from a supply of pressurised gas.

In a preferred embodiment, the actuator is also arranged to open and close the gas supply valve, and the actuator is arranged to open the gas supply valve when it closes the gas vent valve, and vice versa. The shared actuator may be configured to open and close the gas supply valve and gas vent valve substantially simultaneously.

Alternatively, the gas vent valve and gas supply valve may be operated at different times from each other. They may be operated independently by separate actuators. It may be beneficial for the vent valve to remain open momentarily after the supply valve has been opened to allow the supplied gas to flush exhaled air containing carbon dioxide from the mask and tubing.

During an inhalation phase, the system may provide a flow of gas to the user via the gas supply valve while the gas vent valve is closed. During exhalation, the gas supply valve is closed, blocking the supply to the patient whilst the gas vent valve is open, allowing free-flow of exhaled gas away from the patient.

Pressurised gas may be supplied by a substantially silent external air blower for example.

Control of the gas flow in this manner may prevent mixing of clean gas (for inhalation) with exhaled gas which is contaminated with CO₂.

The actuator may be switchable between first and second stable rest positions, and in its first stable position, the gas supply valve is open and the gas vent valve is closed, and in its second stable position, the gas supply valve is closed and the gas vent valve is open.

The actuator(s) may employ Binary Actuation Technology (BAT) as described in WO 2018/046909. Use of a quick-acting actuator may ensure that positive gas flow is provided to the patient during inhalation before their airway starts to collapse and obstruct breathing. This should help prevent both snoring and apnoea from occurring. Use of an actuator as described in WO 2018/046909 can provide substantially silent switching operation so that the patient is not disturbed by noise from the system.

Each stable position of the actuator may be maintained using passive magnetic forces only, that is without the need for power to be supplied to the device, thereby reducing its power consumption. This energy efficiency means that the system may conveniently be powered using a battery supply. Preferably, the actuator is an electromagnetic actuator switchable from one stable position to another by the application of a current pulse to a coil within the actuator.

A gas reservoir may be included in the gas flow path from a supply of pressurised gas to the supply outlet. Gas may be supplied to the user via a bellows or a reservoir. This may provide a delivery profile (in terms of flow rate per second) for the gas which is similar to normal breathing. The reservoir may be configured such that when the gas supply valve is opened to supply gas to the user, little if any further gas needs to be pumped into the reservoir during the inhalation phase. Use of a reservoir may assist with use of a mask without an exhaust port for enabling excess gas to escape. The reservoir may assist the delivery of gas at a rate and volume suitable for a user.

The present disclosure further provides a valve and piston actuation assembly comprising: a valve-piston actuator which is switchable between first and second stable positions; a valve; and a piston slidably located in a cylinder for movement between first and second end-of-travel positions, with the cylinder including two ports which extend from the interior to the exterior of the cylinder and an inlet one way check valve and an outlet one way check valve in fluid communication with a respective port; wherein the valve and piston are coupled to the valve-piston actuator such that: in the first stable position the valve is open and the piston is in its first end-of-travel position, switching the valve-piston actuator towards and into its second stable position switches the valve into a closed position and moves the piston towards and into its second end-of-travel position which urges gas to flow from the interior of the cylinder via the outlet one way check valve, and switching the valve-piston actuator towards and into its first stable position switches the valve back into its open position and moves the piston towards and into its first end-of-travel position which draws gas into the cylinder via the inlet one way check valve.

Such an assembly may be employed to deliver a "puff' of gas according to timing governed by an associated controller. It may be incorporated in a system for controlling a supply of gas for inhalation by a user as described herein. The gas vent valve and the actuator of the system may then be provided by the valve and the valve-piston actuator of the assembly, respectively. Thus, the assembly may be employed to provide a gas vent valve for opening and closing the gas flow path for exhaled gas, whilst the actuator also switches the piston to deliver a pulse or bolus of gas to the user via the supply outlet of the system.

The system may include a gas reservoir in a gas flow path from the outlet one way check valve to a user. This reservoir may be in the form of a bellows. It may act as a smoothing element on the gas supply from the piston pump to provide a more natural flow of gas during inhalation.

Preferably, the gas reservoir has a maximum inflation volume which is adjustable.

The gas flow path of gases exhaled by a user may include a valve or restriction to create a small back pressure to maintain a positive pressure in the airway of the user during exhalation.

The present disclosure also provides a valve and actuator assembly comprising: a valve actuator switchable between first and second stable positions; and a first valve coupled to the valve actuator, such that in the first stable position the first valve is closed, and in the second stable position the first valve is at least partially open, wherein the location of the second stable position is adjustable to adjust the extent to which the first valve is open in the second stable position.

Thus, the assembly facilitates adjustment of the proportion of the flow path through the valve which is open in the second stable position of the actuator. This adjustable valve and actuator assembly may be suitable for use in a wide range of applications to control the flow of any type of liquids and/or gases.

Preferably, the actuator is an electromagnetic actuator. The actuator may be operable to move an armature thereof between the first and second rest positions. Each stable position of the armature of the actuator may be maintained using passive magnetic forces only, that is without requiring power to energise a coil of an electromagnet.

The location of the second stable position may be manually adjustable by a user. The adjustment mechanism may be mechanical, without requiring power to be supplied to the device. The adjustment mechanism may include a scale indicating the extent which the first valve is open when the actuator is in the second stable position.

A first stop may define the second stable position and the location of the first stop relative to the body of the first valve may be adjustable to adjust the extent to which the first valve is open in the second stable position.

The first stop may include a screw thread which is engaged with a complementary screw thread defined by the first valve so that rotation of the first stop adjusts the location of the first stop relative to the first valve.

When the first valve is in the second stable position, a stop surface on the first stop may be an engagement with a first valve surface of the first valve. At least one of the stop surface and the first valve surface may be defined by and part of a component formed of resiliently compressible material.

The valve and actuator assembly may include a second valve coupled to the actuator wherein: in the first stable position, the second valve is at least partially open; and in the second stable position, the second valve is closed, and the location of the first stable position is adjustable to adjust the extent to which the second valve is open in the first stable position.

A second stop may define the first stable position, and the location of the second stop relative to the second valve may be adjustable so as to adjust the extent to which the second valve is open in the first stable position. The second valve and/or the second stop may be configured in a similar manner to that described in relation to the first valve and first stop above.

The valve and actuator assembly may be incorporated in a system for controlling a supply of gas for inhalation by a user as described herein. One of the gas vent valve and the gas supply valve of the system may then be provided by the first valve of the assembly and the actuator on the system provided by the valve actuator of the valve and actuator assembly, respectively.

The system may include a vent flow sensor arranged to monitor the gas pressure and/or flow rate in a vent tube carrying exhaled gas from a user and to output a vent signal to the controller. The controller of the system may be configured to be responsive to a signal outputted by the vent flow sensor. This may enable the controller to monitor a user's breathing pattern to ensure delivery of gas to the user even if apnoea is occurring.

The system may include a supply flow sensor arranged to monitor the gas pressure and/or flow rate in a supply tube carrying gas from the supply outlet to a user and to output a supply signal to the controller. The controller of the system may be a responsive to a signal outputted by the supply flow sensor. In response to the signal, the controller may adjust the timing, pressure profile and/or volume of pressurised gas supplied to the user for the next inhalation and phase and/or the timing of the opening and closing of the gas vent valve during the next exhalation phase.

The controller may control operation of the system having regard to achieving a desired pressure within a breathing mask worn by a user during inhalation and/or exhalation.

The system may include a user mask which is fluidically coupled to a supply tube for carrying gas from the supply outlet and a vent tube for carrying exhaled gas from a user. The mask may have a "dual lumen" tube system. There may be one tube for carrying gas into the mask from the supply source and another separate tube for carrying exhaled gas from the user away from the mask. The system may be configured such that substantially all exhaled CO₂ enters the vent tube or has been expelled into the atmosphere before the next breath is taken. In a preferred embodiment, the mask is a nasal mask. A high proportion of people breathe through their nose during sleep and so this will cater for their needs. The mask (and possibly also some associated tubing) may be a separate disposable component.

The user mask may include a port which is selectively openable to open the mask to the surrounding atmosphere to make it easier for a user to exhale. For example, the port may comprise a closure which is movable between a first position in which it closes an aperture in the mask and a second position in which the aperture is open. The system may include an actuation device for selectively opening the port. The actuation device may comprise an electromagnetic actuator, a piezoelectric actuator, or a hydraulically or pneumatically operated actuator, for example. The system may be arranged to determine when a user is exhaling or about to begin an exhalation, and open the port with reference thereto.

A typical tidal volume for a patient (that is, the volume of each breath) is approximately 500ml and the normal breathing rate is between 12 and 20 breaths per minute. Preferably, the bore of the supply tube and vent tube leading to and from a user's mask is less than 2cm, more preferably less than 1cm and more preferably still around 5mm in diameter. The pressure differences across the tubes in both inhalation and exhalation phases should be sufficiently substantial to allow use of relatively narrow tubes.

The bores of the supply and vent tubes may be different. For example the supply tube may have a wider bore than the vent tube, as for each breath, the exhalation phase may typically be around twice as long as the inhalation phase, leading to a lower flow rate through the vent tube.

Systems as described herein may also be used to implement a patient ventilation system. Ventilation is typically applied to a user invasively through a tracheotomy tube, endotracheal tube, nasotracheal tube or cricothyrotomy tube, or non-invasively using a mask.

Such ventilation systems need to ensure that gas is delivered into the lungs of a patient when every breath is taken. A tracheotomy tube, endotracheal tube, nasotracheal tube or cricothyrotomy tube may be sealed in a trachea with an inflatable balloon. If a mask is to be employed, then this needs to form a gas-tight seal with the face of the patient and cover both the nose and the mouth. If gas is re-circulated within a closed ventilation circuit, a carbon dioxide filter may be included to remove excess carbon dioxide from exhaled gas.

For sleep apnoea applications, a nasal mask is preferred as most users breathe through their nose at night and keep their mouth shut. These masks seal at the nose but the mouth is not covered. Therefore, a sleep apnoea user can open their mouth and breathe at any time, making such a system fail-safe.

In a "closed" ventilation system, the gas flow path from the vent inlet of the system may be coupled to the gas flow path to the supply outlet of the system. More particularly, the vent inlet may be coupled via a vent outlet of the system and a supply inlet of the system back to the supply outlet. Thus a closed gas flow loop may be defined by the system.

The system may include a carbon dioxide filter for filtering carbon dioxide from the gas in the gas flow path to the supply outlet.

It may be preferable to include a humidifier in the system for increasing the humidity of gas in the gas flow path to the supply outlet.

There is further disclosed (but not claimed) a method of controlling a supply of gas for inhalation by a user with a system as described herein comprising the steps of: determining with the controller when a user is about to exhale; opening the gas vent valve; and closing the gas vent valve after the user has exhaled.

The system may be arranged to monitor the gas pressure and/or flow rate in a vent tube carrying exhaled gas from a user, and determine when to open the gas vent valve with reference thereto.

There is further disclosed (but not claimed) a method of controlling a supply of gas for inhalation by a user with a system as described herein comprising the steps of: determining with the controller when a user is due to start the next inhalation; and delivering a volume of gas to the user for the next inhalation.

The system may determine when the user is expected to start to breathe in, in the next inhalation phase, based on the timing of the previous breath. If an apnoea occurs and inhalation does not take place, then the system automatically supplies gas to the patient enabling the airway to open. If the system supplies gas to the patient when it is not expected or required then the user is likely to open their mouth to release excess gas and may wake-up temporarily. The device can then reset and continue to supply gas in time with the user's breathing.

The system may be configured to create a record of the breathing history of a user. A gas flow sensor may be included in the inhalation and/or exhalation gas flow paths in order to provide flow data to the system. The system may record data related to pressure measured at one or more locations in the system and/or movements of valves in the system. Information may be retrieved from the system via a wired connection or wirelessly. It could be made available over the internet for monitoring from a remote location.

The gas supplied to a user may be air drawn from the surrounding atmosphere for example. In some circumstances it may be beneficial to humidify the gas delivered to the user. Although most apnoea sufferers require just air to be delivered during inhalation, some patients may require an oxygen rich gas mixture or substantially pure oxygen to be delivered to maintain the desired oxygen levels in the patient's blood stream.

### Brief description of the drawings

Embodiments of the invention will now be described by way of example and with reference to the accompanying schematic drawings, wherein:
Figure 1 is a graph showing a normal adult breathing pattern;
Figures 2 and 3 are block diagrams of a system according to an embodiment of the present invention in inhalation and exhalation modes, respectively;
Figures 4 and 5 are block diagrams of systems according to a further embodiment of the invention in inhalation and exhalation modes, respectively;
Figures 6 and 7 are cross-sectional perspective side views of a valve and piston actuation assembly comprising an electromagnetic actuator in combination with two valves in first and second stable positions, respectively;
Figures 8 and 9 are cross-sectional perspective side views of a valve and piston assembly comprising an electromagnetic actuator in combination with a valve and a piston pump in first and second stable positions, respectively;
Figures 10 to 12 are cross-sectional perspective side views of a valve and actuator assembly in different configurations;
Figure 13 is a cross-sectional enlarged view of part of the assembly in the configuration shown in Figure 12;
Figures 14 and 15 are diagrams illustrating closed and partially open configurations of the assembly of Figures 10 to 13;
Figure 16 is a graph illustrating partial opening of the valve in the assembly of Figures 10 to 13;
Figures 17 and 18 are block diagrams of two embodiments of patient ventilation systems; and
Figure 19 is a block diagram of a modified version of the system shown in Figures 2 and 3.

### Detailed description of the drawings

Corresponding or similar features in modified and different embodiments are identified using the same reference signs in the drawings.

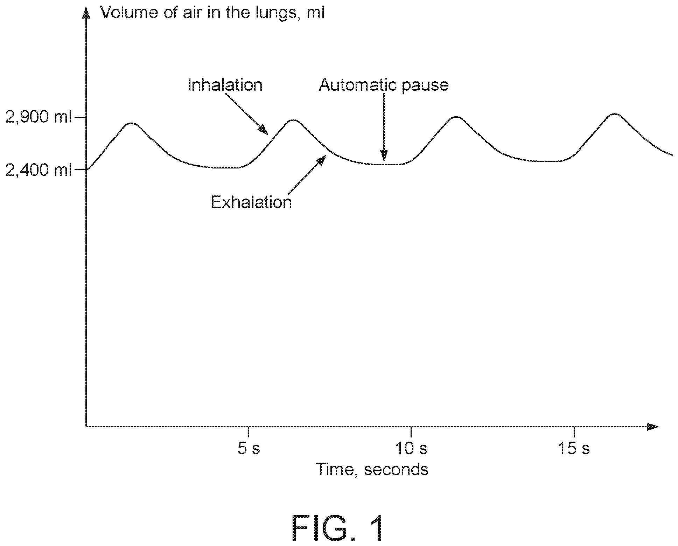

Figure 1 is a graph to illustrate a typical breathing pattern in an adult. The volume of air in the adult's lungs is plotted against time. It can be seen that each complete breath takes approximately five seconds, setting a rate of approximately 12 breaths per minute. Each breath has a total volume of approximately 500ml. Inhalation takes place over approximately one third of each complete breath, exhalation takes place over a further approximately one third and then in the final third, there is little or no flow in or out of the lungs until inhalation starts again. At the end of each breath, a 70kg man retains around 2.4 litres in his lungs.

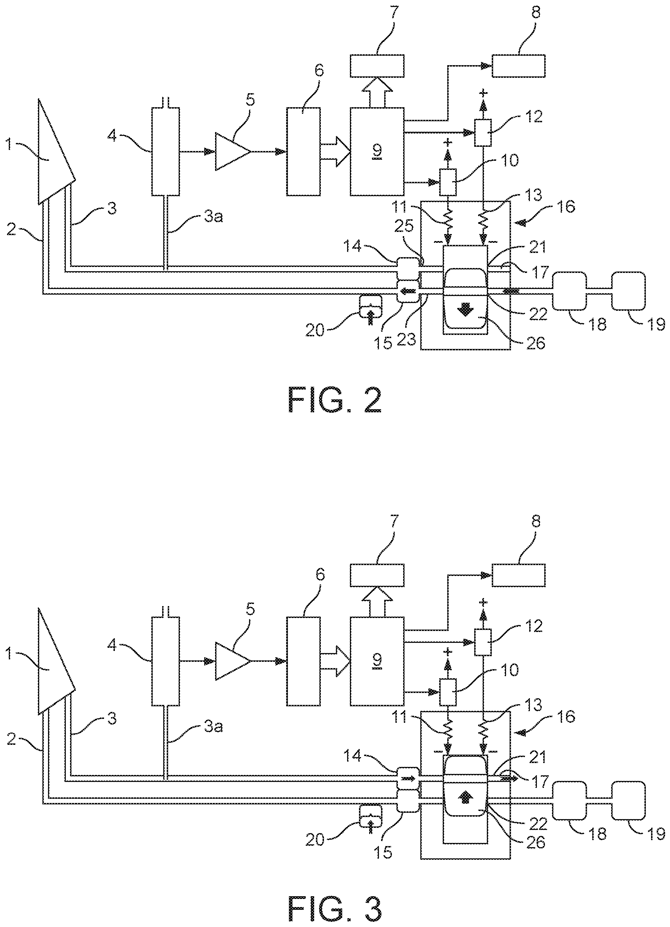

Figures 2 and 3 show an example of the system for controlling supply of gas for inhalation by a user according to the present disclosure. It comprises a breathing attachment 1, such as a mask, with two tubes 2 and 3 coupled to it. Tube 2 is a gas supply tube and tube 3 is a gas vent tube. Each tube may optionally include a respective airflow indicator 15, 14. As a failsafe option, tube 2 may be equipped with a low pressure check valve 20 to allow air to be breathed in even if air is not able to flow into the end of the tube.

A narrow branch tube 3a is coupled to the gas vent tube 3 at one end. The other end of the branch tube 3a is coupled to a high-sensitivity pressure sensor 4. The pressure sensor is connected to an A/D converter 6 via a high gain programmable low pass filter 5. A similar high sensitivity pressure sensor can be coupled to the gas supply tube 2 in a similar manner if required (not shown in the drawing). An output of the A/D converter is coupled to a microcontroller 9. The microcontroller is connected to a small display unit 7 and an optional wireless communication port 8.

A switching assembly 16 opens and closes a gas supply valve 22 and a gas vent valve 21 associated with the supply and vent tubes, respectively. In a first configuration of the switching assembly shown in Figure 1, compressed air is able to flow freely from an air reservoir 18 to a user, via the gas supply valve 22 and a gas supply outlet 23 of the assembly, as the gas supply valve 22 of the switching assembly is open. Air pressure is maintained and constantly supplied in the reservoir by an air pump 19. The pump is preferably a centrifugal air blower. In this configuration, a gas flow path through the gas vent tube 3 and a gas vent inlet 25 of the assembly is blocked by the gas vent valve 21 of the switching assembly. In this way, a constant positive pressure in the breathing attachment 1 can be maintained.

The air reservoir 18 may enable a standard bolus of air to be supplied to a patient for each breath. The air reservoir may be adjustable in volume to enable a bolus to be adjusted for patients with different tidal volumes. The air reservoir may be compliant, like a balloon or bellows, so that the flow of air from the reservoir, when the valve is opened, mimics normal inhalation airflow. The air reservoir may contain a pressure relief valve (not shown) so that when the reservoir is filled with the required volume and pressure of air, any excess air is vented though the pressure relief valve.

A second configuration of the switching assembly is shown in Figure 3. In this case, the assembly allows free flow of air exhaled from the patient's lungs to the surrounding atmosphere via the gas vent valve 21, the vent inlet 25 and a vent outlet 17. The air supply flow from the air reservoir 18 is blocked by gas supply valve 22 of the switching assembly and so positive air pressure is no longer supplied via the supply outlet 23, along the gas supply tube to the breathing attachment 1. Accordingly, it is easier for the patient to breathe out, with the breathing attachment being open to atmospheric pressure. Preferably, the outlet 17 is distant from the patient so that the patient is not disturbed by the flow of air from it.

Switching of an actuator of the switching assembly between its first and second configurations is controlled by the microcontroller 9. The microcontroller is coupled to coils 11 and 13 of the actuator via respective drivers 10 and 12. When coil 11 is energised, the actuator switches its armature 26 into one configuration and coil 13 is energised, it switches into the other configuration. Preferably, the switching assembly includes an actuator having high energy efficiency. The driving signals sent to the coils may be very short, preferably no longer than 10ms.

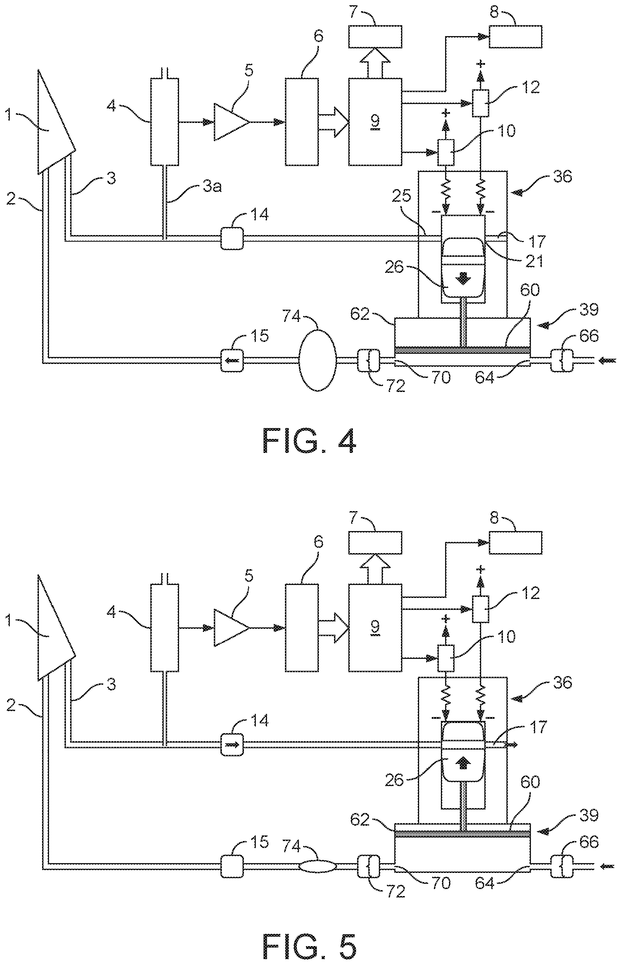

Figures 4 and 5 show another system for controlling a supply of gas for inhalation by a user according to a further embodiment of the invention. A different configuration to that of Figures 2 and 3 is used for delivering a supply of gas for inhalation. The switching assembly 36 operates a gas vent valve 21 for opening and closing a gas flow path from the breathing attachment 1, via the gas vent tube 3, which extends to the surroundings of the system. The switching assembly also operates a piston pump 39. The piston pump comprises a piston 60 slidably mounted in a cylinder 62. The cylinder has an inlet 64 coupled to an inlet one-way check valve 66, which is coupled on the opposite side to the surrounding atmosphere. The cylinder has an outlet 70 coupled to an outlet one-way check valve 72. The other side of the outlet one-way check valve is in fluid communication with a bellows 74 which is in turn coupled to the gas supply tube 2.

In the configuration shown in Figure 4, the actuator armature 26 of the switching assembly has moved into a first stable position. In doing so, it has pushed piston 60 into cylinder 62, causing gas to be expelled from the cylinder via outlet 70 and outlet one-way check valve 72 into the bellows 74. The gas is then delivered by contraction of the bellows into the gas supply tube 2 in a single, discrete puff of gas, promoting air inhalation by the user. In this configuration, the gas vent tube 3 is blocked by the gas vent valve 21 of the switching assembly.

A second switching assembly configuration is shown in Figure 5. In this configuration, the assembly allows gas to flow freely from the gas vent tube via the gas vent valve 21 to the surroundings through outlet 17. As the actuator armature 26 of the switching assembly moves into this configuration, the piston 60 is drawn out of the cylinder. This in turn causes air from the surrounding atmosphere to be drawn into the cylinder via the inlet one-way check valve 66. The air supply flow from the empty bellows 37 has stopped and therefore does not inhibit the user's ability to exhale.

The check valves 66 and 72 may act as an emergency air supply path in case of pump malfunction.

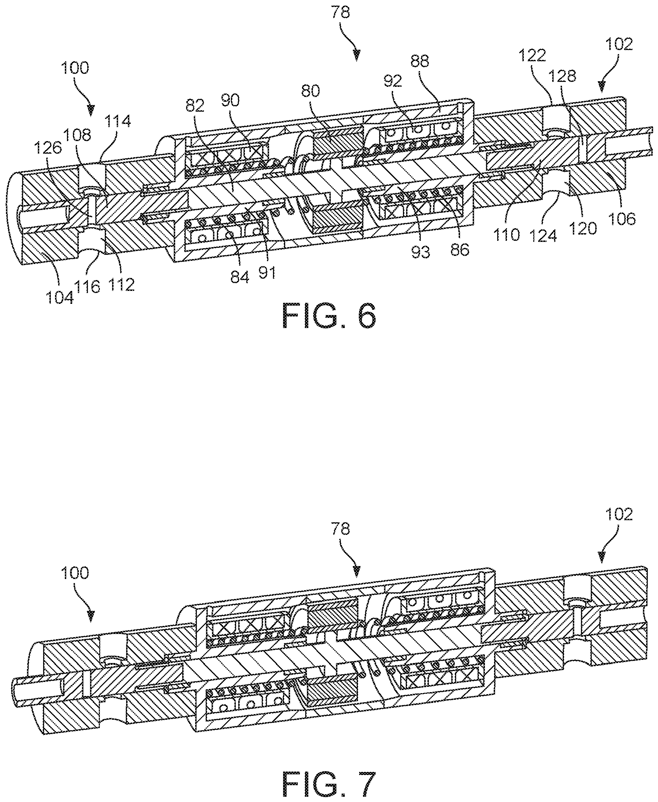

Figures 6 and 7 show an actuator 78 in combination with two valves suitable for use in the system of Figures 2 and 3. The actuator includes an armature comprising a radially polarised annular magnet 80 which is attached to a push-pull rod 82. Two springs 84 and 86 are axially aligned with and extend around the push-pull rod and are located at respective ends of the actuator housing 88. A pair of coils 90 and 92 are coaxially located around the push-pull rod and respective coil cores 91 and 93 at opposite ends of the housing. In operation of the actuator, energising one of the coils attracts the magnet 80 towards one end of its range of travel, into a stable rest position held by magnetic flux flowing from the permanent magnet 80 around the housing. In this position, an adjacent spring is compressed, providing mechanical energy storage. When the other coil is energised, the magnet is switched to its other stable rest position at the other end of its range of travel.

Two on-off valve assemblies 100, 102 are mounted on the actuator housing at axially opposite ends. Each valve assembly comprises a valve body 104, 106 within which a respective plunger 108, 110 is slidably located. The plungers are fixedly coupled to opposite ends of the push-pull rod 82.

Valve body 104 defines a channel 112 therethrough, which extends between a pair of ports 114, 116. Similarly, valve body 102 defines a channel 120, which extends between a pair of ports 122 and 124. Plunger 108 defines a conduit 126 which extends perpendicular to its longitudinal axis and plunger 110 defines a conduit 128, also perpendicular to its longitudinal axis.

In a first stable position of the actuator shown in Figure 6, conduit 128 is displaced from channel 120 and so valve assembly 102 is in its closed configuration, whilst conduit 126 is aligned with ports 114 and 116 so that valve assembly 100 is in its open configuration.

Conversely, in the second stable configuration of the actuator shown in Figure 7, valve assembly 102 is in its open configuration, whilst assembly 100 is in its closed configuration.

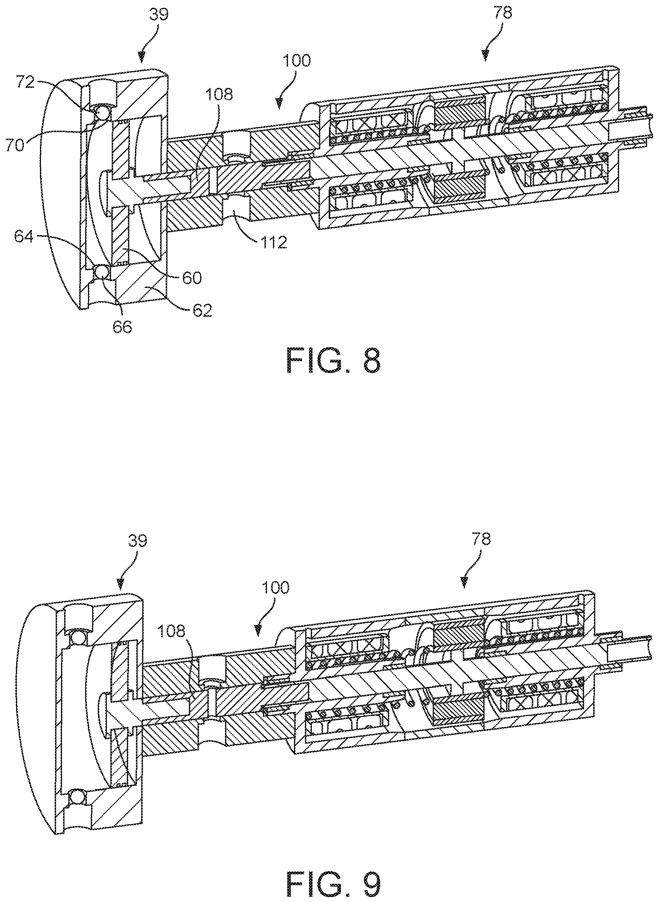

A switching assembly suitable for use in the system depicted in Figures 4 and 5 is shown in Figures 8 and 9. The actuator 78 and valve assembly 100 correspond to those shown in Figures 6 and 7. Valve assembly 102 is omitted. Instead, the piston 60 of a piston pump 39 is coupled to the distal end of plunger 108. The configuration shown in Figure 8 corresponds to that depicted schematically in Figure 4, whilst that of Figure 9 corresponds to that depicted in Figure 5.

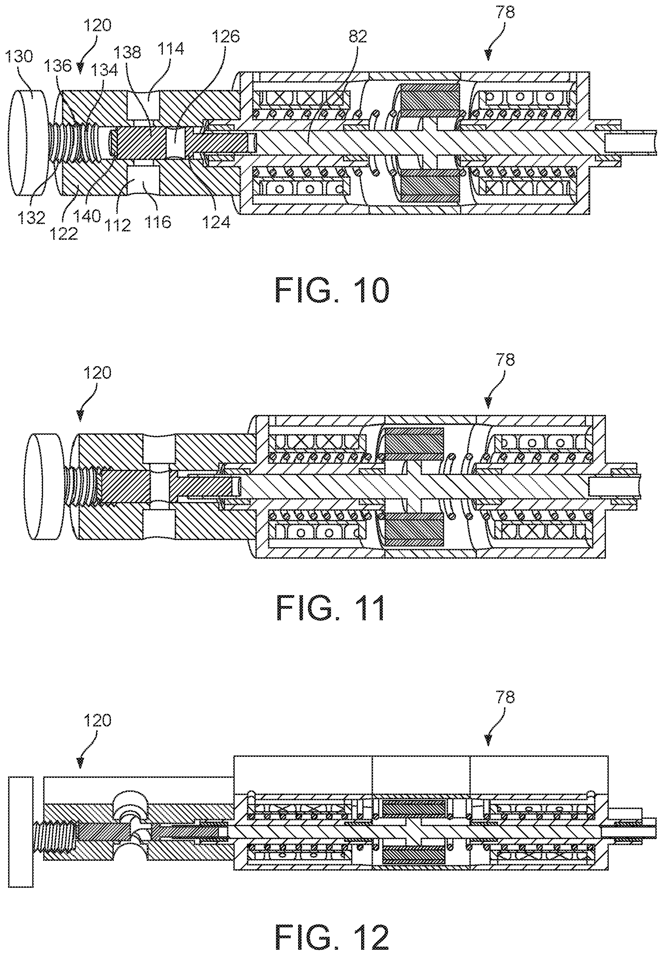

A further valve and actuator assembly for use in systems described herein will now be described with reference to Figures 10 to 16. An actuator 78 similar to those described above is provided in combination with an adjustable valve assembly 120. The valve assembly 120 is mounted on the actuator housing at one end thereof. A further adjustable valve assembly may similarly be mounted on the axially opposite end of the housing for operation by the actuator in a similar manner to the two valve-and-actuator combination shown in Figures 6 and 7.

The valve assembly 120 comprises a valve body 122 within which a plunger 124 is slidably located. The plunger is fixedly coupled to one end of the push-pull rod 82 of the actuator. Valve body 122 defines a channel 112 therethrough, which extends between a pair of ports 114 and 116. Plunger 124 defines a conduit 126 which extends perpendicular to its longitudinal axis.

A stop 130 carries a screw thread 132 which is in engagement with a complementary screw thread 134 defined by the valve body 122. The screw thread is coaxial with the plunger 124 of the valve assembly so that rotation of the stop relative to the valve body moves a stop surface 136 of the stop towards or away from a distal end 138 of the plunger 124. A bumper 140 is located on the distal end of the plunger. The bumper is formed of a resiliently compressible material and defines a valve surface for engagement with the stop surface of the stop 130.

The resilient bumper may be included to minimise any sound generated as the plunger moves into contact with the stop, which may be particularly desirable in medical applications to avoid disturbing a user.

Figure 10 depicts a first stable position of the actuator. Conduit 126 is displaced from channel 112 and so the valve assembly 120 is in its closed configuration.

In a second stable configuration of the actuator shown in Figure 11, the valve assembly 120 is in its open configuration, with conduit 126 aligned with the channel 112.

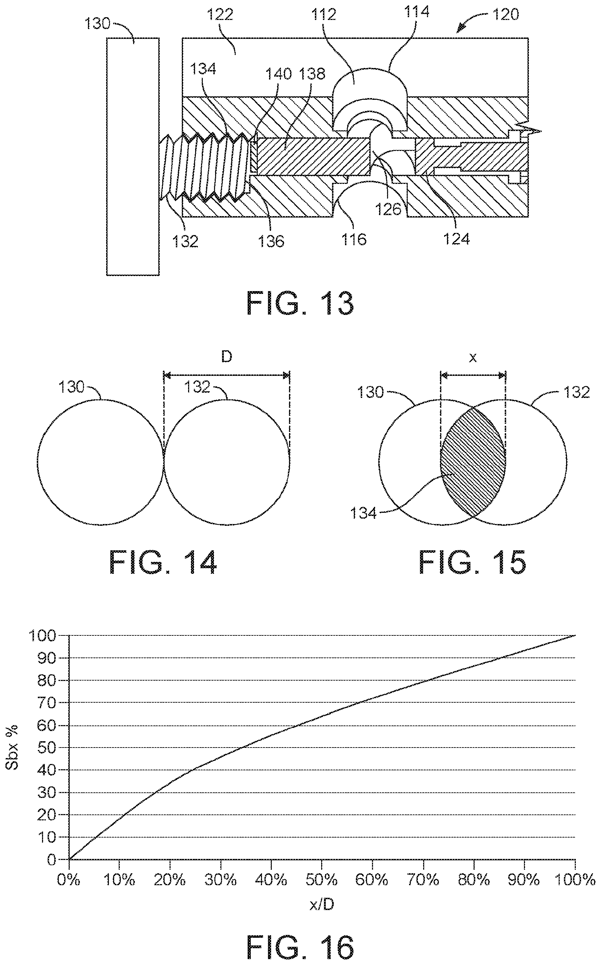

In Figure 12, the stop 130 has been rotated to screw it further into the valve assembly body 122 so that it engages the distal end 138 of the plunger 124 before it moves into the fully opened position showed in Figure 11. Accordingly, by altering the position of the stop 130, it is possible to adjust the extent to which the valve is open when the actuator is in its second stable configuration. The valve is still held in this partly open configuration by the actuator by means of passive magnetic forces within the actuator and so the power consumption of the actuator is still minimised. The stop therefore acts as a flow adjuster to alter the cross-sectional area of the flow path through the valve assembly. This operation is illustrated further by Figures 14 to 16.

In Figures 14 and 15, the cross-section of the channel 112 through the valve assembly adjacent to the plunger 124 is represented by circular shape 130, whilst a cross-section of the conduit 126 through the plunger 124 is represented by circular shape 132, each having a diameter "D". In Figure 14, there is no overlap between the shapes 130 and 132, representing displacement of the conduit 126 axially from the channel 112. In Figure 15, the plunger has been moved relative to the valve body 122 such that shape 132 partially overlaps with shape 130, to define an opening 134 through the valve assembly having a width in the axial direction of "x". In Figure 14, the valve is closed, whilst in the Figure 15 configuration, gas flow is permitted through the valve assembly, to a restricted extent in comparison with when the valve is fully opened.

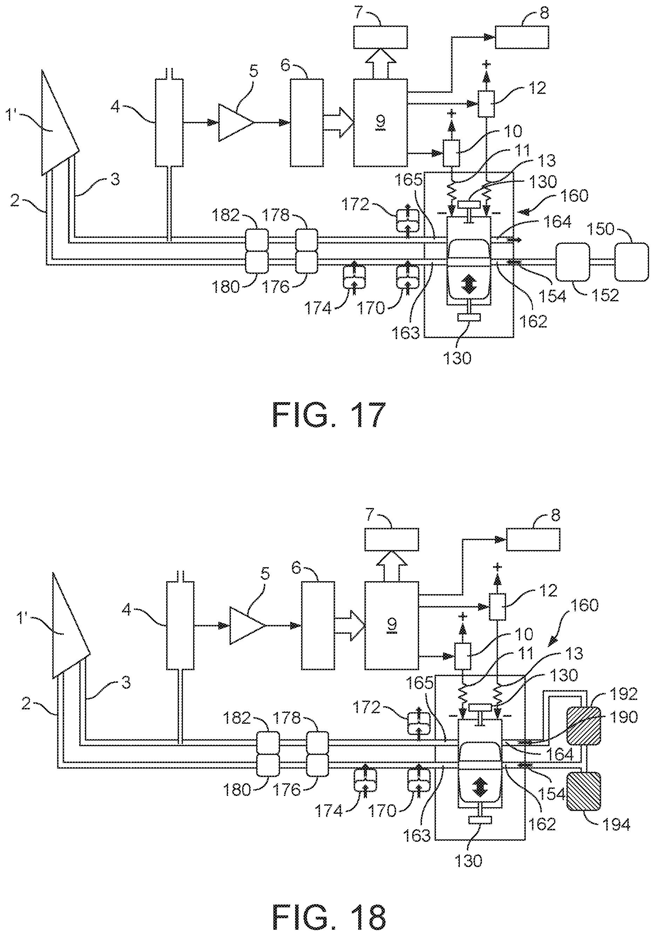

Figure 16 is a graph showing the relationship between the extent to which the valve is open, measured as x as a percentage of D, plotted against "Sbx". Sbx is a measure of the extent to which the valve is open as a percentage of its fully opened configuration, including an estimation of the effects of boundary layers in the gas flow through the open cross-section 134. Figures 17 and 18 show embodiments of a patient ventilation system according to the present disclosure.

Figure 17 shows a forced breathing system, whilst Figure 18 shows a closed breathing system. A mask 1' is designed to form a seal around both the nose and mouth of the patient. Alternatively, the system could be connected to a tracheotomy tube, endotracheal tube, nasotracheal tube or cricothyrotomy tube.

In the system of Figure 17, a source of pressurised gas 150 is fluidically coupled to a gas humidifier 152. The output of the humidifier is coupled to a supply inlet 154 of a valve and actuator assembly 160. The assembly 160 comprises an actuator 78 in combination with a valve assembly 120 as shown in Figures 10 to 13, further modified to include another valve assembly coupled to the opposite end of the actuator. Thus, each valve assembly is adjustable using a respective stop 130 to alter the extent to which the valve is open in its open configuration.

The assembly 160 includes a gas supply valve 162 and a gas vent valve 164 associated with the supply tube 2 and vent tube 3, respectively. In a first configuration of the assembly shown in Figure 17, gas is able to flow from the inlet 154 of the assembly through valve 162, and supply outlet 163. In this configuration, a gas flow path via the gas vent tube 3 and a vent inlet 165 of the assembly is blocked by the gas vent valve 164 of the assembly. This ensures that a positive pressure can be obtained in the mask 1'.

In a similar manner to the valve switching assemblies of Figures 2 and 3, the assembly 160 can be switched from the configuration shown in Figure 17 to a second stable configuration by the actuator, in which gas supply valve 162 is closed and gas vent valve 164 is open.

The gas supply tube 2 includes an inhale safety check valve 170 and the gas vent tube 3 includes an exhale safety check valve 172. These check valves ensure that a patient cannot be prevented by the system from breathing in or out.

An inlet port 174 is provided in the gas supply tube 2 to allow other medically active gases to be selectively included in the gas supplied to a patient. The gas supply tube 2 and gas vent tube 3 may also include respective gas flow indication devices 176 and 178.

In addition, the gas supply tube 2 and gas vent tube 3 may include respective tube couplings 180 and 182 at locations spaced from the mask 1' to enable removal of a used mask assembly and its replacement with a fresh mask assembly.

In the closed ventilation system of Figure 18, the assembly has a vent outlet 190 for receiving exhaled gas from the gas vent tube 3 via the valve and actuator assembly 160. The outlet 190 is fluidically coupled to a carbon dioxide filtering device 192. After passing through this device, gas is fed back to the inlet 154 via a return tube 196. A resilient air reservoir 194 may also be fluidically coupled to tube 196.

The gas flowing in the ventilation systems may be air. It may also be air combined with additional oxygen or other medically active substances for example.

As with the systems depicted in Figures 2 and 3, switching of the actuator of the assembly 160 between its first and second stable positions is controlled by the microcontroller 9 in response to the gas pressure and/or flow rate detected in the gas vent tube 3. The system is thereby able to monitor the breathing rate of the patient and supply pressurised gas to the mask 1' in order to maintain breathing by the patient at an appropriate rate in the embodiment of Figure 17. In the embodiment of Figure 18, gas may be circulated through the system by the patient's lungs and the switching of the assembly 160 is controlled to be synchronised with the patient's breathing. If a patient is fully anaesthetised, the system may be configured to provide a selected breathing rate and tidal volume for the patient automatically.

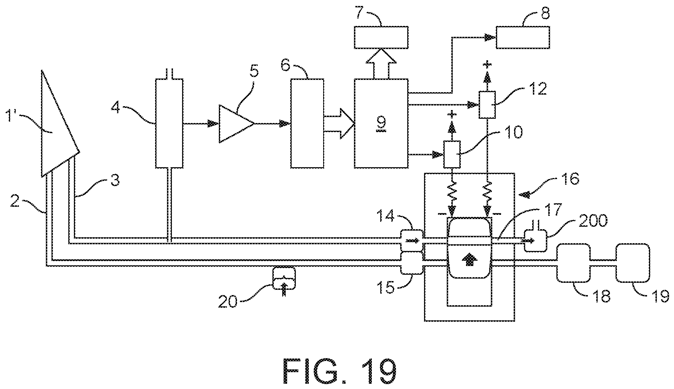

Figure 19 shows a modified version of the system shown in Figures 2 and 3. The modified system additionally includes a gas pressure reduction device 200 coupled to the vent gas flow path for carrying exhaled gas from a user. As shown in Figure 19, the gas pressure reduction device may be fluidically coupled to the vent outlet 17 of switching assembly 16. The gas pressure reduction device may be in the form of an electrically powered pump. It is arranged to reduce the gas pressure in the gas vent tube 3 and so draw exhaled gas along the tube as the user exhales in order to assist the user in exhaling and to encourage evacuation of exhaled carbon dioxide from the mask.

## Claims

1. A system for controlling a supply of gas for inhalation by a user comprising:
a supply outlet (23) for supplying gas to a user;
a vent inlet (25) for receiving exhaled gas from a user; and
a gas vent valve (21) for opening and closing a gas flow path from the vent inlet, **characterised in that**
the system includes:
an electromagnetic actuator arranged to open and close the gas vent valve (21); and
a controller (9) for controlling operation of the actuator, and
the actuator comprises:
an armature (26) comprising a permanent magnet (80);
two coils (11,13; 90,92); and
two coil cores (91,93), each of which extends within a respective coil;
wherein the armature (26) is moveable relative to the coils (11,13; 90,92) between first and second stable rest positions by passing a current through at least one of the coils, and
in each rest position, the armature is closer to one of the coil cores than the other of the coil cores and is spaced by a gap from the closer coil core.

2. A system of claim 1 including a gas pressure reduction device (200) coupled to a vent gas flow path carrying exhaled gas from a user to reduce the gas pressure in the vent gas flow path between the user and the device, wherein preferably the gas pressure reduction device is located downstream of the gas vent valve (21).

3. A system of claim 1 or claim 2, wherein the actuator includes two resilient components (84,86), wherein one of the resilient components is compressed by movement of the armature towards each stable rest position, and preferably one of the resilient components exerts a force on the armature as it moves into each rest position which prevents the armature from contacting the coil core that it is moving towards.

4. A system of any preceding claim, including a gas supply valve (22) for opening and closing a gas flow path to the supply outlet (23), wherein preferably the actuator is also arranged to open and close the gas supply valve, and the actuator is arranged to open the gas supply valve when it closes the gas vent valve (21), and vice versa, preferably in the first stable position, the gas supply valve (22) is open and the gas vent valve (21) is closed, and in its second stable position, the gas supply valve (22) is closed and the gas vent valve (21) is open, and preferably the system includes an air reservoir (18) in the gas flow path from a supply of pressurised gas to the supply outlet

5. A system of any preceding claim, wherein in the first stable position the gas vent valve (21) is closed, and
in the second stable position the gas vent valve is at least partially open, wherein the location of the second stable position is adjustable to adjust the extent to which the gas vent valve is open in the second stable position.

6. A system of claim 5, wherein the location of the second stable position is manually adjustable.

7. A system of claim 5 or claim 6, wherein a first stop (130) defines the second stable position and the location of the first stop relative to the gas vent valve (21) is adjustable to adjust the extent to which the gas vent valve is open in the second stable position, preferably the first stop includes a screw thread (132) which is engaged with a complementary screw thread (134) so that rotation of the first stop adjusts the location of the first stop relative to the gas vent valve, and preferably a stop surface (136) of the first stop engages a gas vent valve surface of the gas vent valve when the gas vent valve is in the second stable position, and at least one of the stop surface and the gas vent valve surface is defined by resiliently compressible material.

8. A system of any of claims 5 to 7 including a second valve coupled to the actuator, wherein:
in the first stable position, the second valve is at least partially open; and
in the second stable position, the second valve is closed, and the location of the first stable position is adjustable to adjust the extent to which the second valve is open in the first stable position, and preferably a second stop defines the first stable position, and the location of the second stop relative to the second valve is adjustable so as to adjust the extent to which the second valve is open in the first stable position.

9. A system of claim 8, wherein the gas supply valve of the system is provided by the second valve of the assembly.

10. A system of any preceding claim including a vent flow sensor arranged to monitor the gas pressure and/or flow rate in a vent tube carrying exhaled gas from a user and to output a vent signal to the controller.

11. A system of any preceding claim including a supply flow sensor arranged to monitor the gas pressure and/or flow rate in a supply tube carrying gas from the supply outlet to a user and to output a supply signal to the controller.

12. A system of any preceding claim including a user mask (1) which is fluidically coupled to a supply tube for carrying gas from the supply outlet and a vent tube for carrying exhaled gas from a user.

13. A system of any preceding claim, wherein the gas flow path from the vent inlet is coupled to the gas flow path to the supply outlet.

14. A system of any preceding claim including a carbon dioxide filter (192) for filtering carbon dioxide from gas in the gas flow path to the supply outlet.

15. A system of any preceding claim including a humidifier (152) for increasing the humidity of gas in the gas flow path to the supply outlet.

## Patentansprüche

1. System zum Steuern einer Gaszufuhr zum Inhalieren durch einen Benutzer, umfassend:
einen Zufuhrauslass (23) zum Zuführen von Gas zu einem Benutzer;
einen Entlüftungseinlass (25) zum Aufnehmen von ausgeatmetem Gas von einem Benutzer; und
ein Gasentlüftungsventil (21) zum Öffnen und Schließen eines Gasströmungswegs vom Entlüftungseinlass, **dadurch gekennzeichnet, dass**
das System Folgendes einschließt:
einen elektromagnetischen Aktuator, dazu angeordnet, das Gasentlüftungsventil (21) zu öffnen und zu schließen; und
eine Steuerung (9) zum Steuern des Betriebs des Aktuators, und
wobei der Aktuator Folgendes umfasst:
einen Anker (26), umfassend einen Permanentmagneten (80) ;
zwei Spulen (11, 13; 90, 92); und
zwei Spulenkerne (91, 93), von denen sich jeder innerhalb einer jeweiligen Spule erstreckt;
wobei der Anker (26) relativ zu den Spulen (11, 13; 90, 92) zwischen einer ersten und einer zweiten stabilen Ruheposition bewegbar ist, indem ein Strom durch mindestens eine der Spulen geleitet wird, und
wobei der Anker sich in jeder Ruheposition näher an einem der Spulenkerne als an dem anderen der Spulenkerne befindet und durch einen Spalt von dem näheren Spulenkern beabstandet ist.

2. System nach Anspruch 1, einschließlich einer Gasdruckverringerungsvorrichtung (200), die mit einem Entlüftungsgasströmungsweg gekoppelt ist, der ausgeatmetes Gas von einem Benutzer führt, um den Gasdruck in dem Entlüftungsgasströmungsweg zwischen dem Benutzer und der Vorrichtung zu verringern, wobei die Gasdruckverringerungsvorrichtung vorzugsweise stromabwärts des Gasentlüftungsventils (21) angeordnet ist.

3. System nach Anspruch 1 oder Anspruch 2, wobei der Aktuator zwei elastische Komponenten (84, 86) einschließt, wobei eine der elastischen Komponenten durch die Bewegung des Ankers in Richtung jeder stabilen Ruheposition zusammengedrückt wird und vorzugsweise eine der elastischen Komponenten eine Kraft auf den Anker ausübt, wenn er sich in jede Ruheposition bewegt, wodurch verhindert wird, dass der Anker den Spulenkern berührt, auf den er sich zubewegt.

4. System nach einem der vorhergehenden Ansprüche, einschließlich eines Gaszufuhrventils (22) zum Öffnen und Schließen eines Gasströmungswegs zum Zufuhrauslass (23), wobei vorzugsweise der Aktuator auch dazu angeordnet ist, das Gaszufuhrventil und den Aktuator zu öffnen und zu schließen, und der Aktuator dazu angeordnet ist, das Gaszufuhrventil zu öffnen, wenn es das Gasentlüftungsventil (21) schließt, und umgekehrt, wobei vorzugsweise in der ersten stabilen Position, das Gaszufuhrventil (22) offen ist und das Gasentlüftungsventil (21) geschlossen ist, und in seiner zweiten stabilen Position das Gaszufuhrventil (22) geschlossen ist und das Gasentlüftungsventil (21) offen ist, und vorzugsweise wobei das System ein Luftreservoir (18) in dem Gasströmungsweg von einer Druckgaszufuhr zu dem Zufuhrauslass enthält.

5. System nach einem der vorhergehenden Ansprüche, wobei in der ersten stabilen Position das Gasentlüftungsventil (21) geschlossen ist und
in der zweiten stabilen Position ist das Gasentlüftungsventil zumindest teilweise geöffnet ist, wobei die Lage der zweiten stabilen Position einstellbar ist, um das Ausmaß einzustellen, in dem das Gasentlüftungsventil in der zweiten stabilen Position geöffnet ist.

6. System nach Anspruch 5, wobei die Lage der zweiten stabilen Position manuell einstellbar ist.

7. System nach Anspruch 5 oder Anspruch 6, wobei ein erster Anschlag (130) die zweite stabile Position definiert und die Lage des ersten Anschlags relativ zu dem Gasentlüftungsventil (21) einstellbar ist, um das Ausmaß einzustellen, in dem das Gasentlüftungsventil in der zweiten stabilen Position offen ist, wobei vorzugsweise der erste Anschlag ein Schraubgewinde (132) einschließt, das mit einem komplementären Schraubgewinde (134) in Eingriff steht, sodass eine Drehung des ersten Anschlags die Lage des ersten Anschlags relativ zu dem Gasentlüftungsventil einstellt, und vorzugsweise eine Anschlagsoberfläche (136) des ersten Anschlags eine Gasentlüftungsventiloberfläche des Gasentlüftungsventils in Eingriff nimmt, wenn sich das Gasentlüftungsventil in der zweiten stabilen Position befindet, und mindestens eine von der Anschlagsoberfläche und der Gasentlüftungsventiloberfläche durch elastisch komprimierbares Material definiert ist.

8. System nach einem der Ansprüche 5 bis 7, einschließlich eines zweiten Ventils, das mit dem Aktuator gekoppelt ist, wobei:
in der ersten stabilen Position das zweite Ventil zumindest teilweise geöffnet ist; und
in der zweiten stabilen Position das zweite Ventil geschlossen ist und die Lage der ersten stabilen Position einstellbar ist, um das Ausmaß einzustellen, in dem das zweite Ventil in der ersten stabilen Position geöffnet ist, und vorzugsweise ein zweiter Anschlag die erste stabile Position definiert, und die Lage des zweiten Anschlags in Bezug auf das zweite Ventil einstellbar ist, um das Ausmaß einzustellen, in dem das zweite Ventil in der ersten stabilen Position geöffnet ist.

9. System nach Anspruch 8, wobei das Gaszufuhrventil des Systems durch das zweite Ventil der Anordnung bereitgestellt wird.

10. System nach einem der vorhergehenden Ansprüche, einschließlich eines Entlüftungsströmungssensors, dazu angeordnet, den Gasdruck und/oder die Strömungsrate in einem Entlüftungsrohr zu überwachen, das ausgeatmetes Gas von einem Benutzer führt, und ein Entlüftungssignal an die Steuerung auszugeben.

11. System nach einem der vorhergehenden Ansprüche, einschließlich eines Zufuhrströmungssensors, dazu angeordnet, den Gasdruck und/oder die Strömungsrate in einem Zufuhrrohr zu überwachen, das Gas von dem Zufuhrauslass zu einem Benutzer führt, und ein Zufuhrsignal an die Steuerung auszugeben.

12. System nach einem der vorhergehenden Ansprüche, einschließlich einer Benutzermaske (1), die mit einem Zufuhrrohr zum Führen von Gas von dem Zufuhrauslass und einem Entlüftungsrohr zum Führen von ausgeatmetem Gas von einem Benutzer fluidisch gekoppelt ist.

13. System nach einem der vorhergehenden Ansprüche, wobei der Gasströmungsweg vom Entlüftungseinlass mit dem Gasströmungsweg zum Zufuhrauslass gekoppelt ist.

14. System nach einem der vorhergehenden Ansprüche einschließlich eines Kohlendioxidfilters (192) zum Filtern von Kohlendioxid aus Gas im Gasströmungsweg zum Zufuhrauslass.

15. System nach einem der vorhergehenden Ansprüche einschließlich eines Befeuchters (152) zum Erhöhen der Feuchtigkeit des Gases im Gasströmungsweg zum Zufuhrauslass.

## Revendications

1. Système de commande d'alimentation en gaz pour inhalation par un utilisateur comprenant :
une sortie d'alimentation (23) pour fournir du gaz à un utilisateur ;
une entrée de ventilation (25) pour recevoir du gaz exhalé par un utilisateur ; et
une valve de ventilation de gaz (21) pour ouvrir et fermer un trajet d'écoulement de gaz depuis l'entrée de ventilation,
**caractérisé en ce que**
le système comprend :
un actionneur électromagnétique agencé pour ouvrir et fermer la valve de ventilation de gaz (21) ; et
un contrôleur (9) pour commander le fonctionnement de l'actionneur, et
l'actionneur comprend :
une armature (26) comprenant un aimant permanent (80) ;
deux bobines (11, 13 ; 90, 92) ; et
deux noyaux de bobine (91, 93), chacun s'étendant à l'intérieur d'une bobine respective ;
dans lequel l'armature (26) est mobile par rapport aux bobines (11, 13 ; 90, 92) entre des première et seconde positions de repos stables par le passage d'un courant à travers au moins l'une des bobines, et
dans chaque position de repos, l'armature est plus proche de l'un des noyaux de bobine que de l'autre des noyaux de bobine et est espacée d'un intervalle du noyau de bobine plus proche.

2. Système selon la revendication 1, comprenant un dispositif de réduction de pression de gaz (200) couplé à un trajet d'écoulement de gaz de ventilation transportant du gaz exhalé par un utilisateur pour réduire la pression de gaz dans le trajet d'écoulement de gaz de ventilation entre l'utilisateur et le dispositif, dans lequel de préférence le dispositif de réduction de pression de gaz est situé en aval de la valve de ventilation de gaz (21).

3. Système selon la revendication 1 ou la revendication 2, dans lequel l'actionneur comprend deux composants élastiques (84, 86), dans lequel l'un des composants élastiques est comprimé par le déplacement de l'armature en direction de chaque position de repos stable, et de préférence l'un des composants élastiques exerce une force sur l'armature lorsqu'elle se déplace dans chaque position de repos, ce qui empêche l'armature d'entrer en contact avec le noyau de bobine vers lequel elle se déplace.

4. Système selon l'une quelconque des revendications précédentes, comprenant une valve d'alimentation en gaz (22) pour ouvrir et fermer un trajet d'écoulement de gaz vers la sortie d'alimentation (23), dans lequel de préférence l'actionneur est également agencé pour ouvrir et fermer la valve d'alimentation en gaz, et l'actionneur est agencé pour ouvrir la valve d'alimentation en gaz lorsqu'il ferme la valve de ventilation de gaz (21), et vice versa, de préférence dans la première position stable, la valve d'alimentation en gaz (22) est ouverte et la valve de ventilation de gaz (21) est fermée, et dans sa seconde position stable, la valve d'alimentation en gaz (22) est fermée et la valve de ventilation de gaz (21) est ouverte, et de préférence le système comprend un réservoir d'air (18) dans le trajet d'écoulement de gaz depuis une alimentation en gaz sous pression vers la sortie d'alimentation.

5. Système selon l'une quelconque des revendications précédentes, dans lequel dans la première position stable la valve de ventilation de gaz (21) est fermée, et
dans la seconde position stable la valve de ventilation de gaz est au moins en partie ouverte, dans lequel l'emplacement de la seconde position stable est réglable pour régler l'étendue de l'ouverture de la valve de ventilation de gaz dans la seconde position stable.

6. Système selon la revendication 5, dans lequel l'emplacement de la seconde position stable est réglable manuellement.

7. Système selon la revendication 5 ou la revendication 6, dans lequel une première butée (130) définit la seconde position stable et l'emplacement de la première butée par rapport à la valve de ventilation de gaz (21) est réglable pour régler l'étendue de l'ouverture de la valve de ventilation de gaz dans la seconde position stable, de préférence la première butée comprend un filetage (132) qui est en prise avec un filet complémentaire (134) afin que la rotation de la première butée règle l'emplacement de la première butée par rapport à la valve de ventilation de gaz, et de préférence une surface de butée (136) de la première butée vient en prise avec une surface de valve de ventilation de gaz de la valve de ventilation de gaz lorsque la valve de ventilation de gaz est dans la seconde position stable, et au moins l'une de la surface de butée et de la surface de valve de ventilation de gaz est définie par un matériau compressible élastique.

8. Système selon l'une quelconque des revendications 5 à 7, comprenant une seconde valve couplée à l'actionneur, dans lequel :
dans la première position stable, la seconde valve est au moins en partie ouverte ; et
dans la seconde position stable, la seconde valve est fermée, et l'emplacement de la première position stable est réglable pour régler l'étendue de l'ouverture de la seconde valve dans la première position stable, et de préférence une seconde butée définit la première position stable, et l'emplacement de la seconde butée par rapport à la seconde valve est réglable afin de régler l'étendue de l'ouverture de la seconde valve dans la première position stable.

9. Système selon la revendication 8, dans lequel la valve d'alimentation en gaz du système est fournie par la seconde valve de l'ensemble.

10. Système selon l'une quelconque des revendications précédentes, comprenant un capteur d'écoulement de ventilation agencé pour surveiller la pression de gaz et/ou le débit de gaz dans un tube de ventilation transportant le gaz exhalé par un utilisateur et pour émettre un signal de ventilation au contrôleur.

11. Système selon l'une quelconque des revendications précédentes, comprenant un capteur d'écoulement d'alimentation agencé pour surveiller la pression de gaz et/ou le débit de gaz dans un tube d'alimentation transportant le gaz de la sortie d'alimentation à un utilisateur et pour émettre un signal d'alimentation au contrôleur.

12. Système selon l'une quelconque des revendications précédentes, comprenant un masque d'utilisateur (1) qui est couplé fluidiquement à un tube d'alimentation pour transporter du gaz depuis la sortie d'alimentation et à un tube de ventilation pour transporter le gaz exhalé par un utilisateur.

13. Système selon l'une quelconque des revendications précédentes, dans lequel le trajet d'écoulement de gaz depuis l'entrée de ventilation est couplé au trajet d'écoulement de gaz vers la sortie d'alimentation.

14. Système selon l'une quelconque des revendications précédentes, comprenant un filtre à dioxyde de carbone (192) pour filtrer le dioxyde de carbone du gaz dans le trajet d'écoulement de gaz vers la sortie d'alimentation.

15. Système selon l'une quelconque des revendications précédentes, comprenant un humidificateur (152) pour augmenter l'humidité du gaz dans le trajet d'écoulement de gaz vers la sortie d'alimentation.
